# FASCICULE DE BREVET EUROPEEN

(11) **EP 2 599 472 B1**
(45) Date de publication et mention de la délivrance du brevet: **02.11.2016**
(21) Numéro de dépôt: 12176642.2
(22) Date de dépôt: 17.07.2012
(51) Int. Cl.: A61K 8/34, A61K 8/35, A61K 8/37, A61K 8/92, A61Q 13/00, A61K 8/891

(54) **Solution parfumante stabilisée vis-à-vis des rayons ultraviolets**
Parfümierende Lösung, die gegenüber UV-Strahleneinwirkung stabilisiert ist
Perfuming solution stabilized with respect to ultraviolet radiation

(30) Priorité: 21.07.2011 FR 1156642
(43) Date de publication de la demande: 05.06.2013
(73) Titulaire: LvmH Recherche, 45800 Saint-Jean De Braye (FR)
(72) Inventeur: Germaneau, Sylvie, 45800 Saint Jean de Braye (FR); Alard, Valérie, 45000 Orleans (FR); Perrier, Eric, 38138 Les Cotes d'Arey (FR); Guyot, Maud, 45110 Châteauneuf sur Loire (FR)
(74) Mandataire: Chantraine, Sylvie Hélène

(56) Documents cités:
- EP-A2- 2 324 819
- FR-A1- 2 915 387
- FR-A1- 2 923 385
- DATABASE GNPD [Online] mintel; octobre 2008 (2008-10), "Rain Water Body Mist", XP002678133, Database accession no. 993834

## Description

L'invention se rapporte à une solution parfumante comprenant un parfum stabilisé par un mélange de deux filtres UV particuliers solubilisés dans une huile non volatile.

### Etat de l'art

Les solutions parfumantes se présentent généralement sous la forme d'une eau fraîche, d'une eau de toilette, d'une eau de parfum, d'un parfum, d'une lotion après rasage, ou d'une eau de soin.

Elles sont transparentes et conditionnées dans des flacons eux aussi transparents, si bien que ces produits sont particulièrement exposés à la lumière, aux rayons ultraviolets (également dénommés « rayons UV » ou « UV » dans la présente demande) et aux variations de température.

Or la lumière du jour et les rayons UV provoquent une dégradation des molécules telles que les parfums et les colorants que ces solutions contiennent. On peut observer en particulier un changement du parfum, une décoloration, des jaunissements, des rosissements, des floculations et des précipités de matières, ou des troubles pouvant nuire à la perception du produit par le consommateur et à l'efficacité des pompes, lorsqu'elles sont conditionnées en spray.

Pour protéger les solutions parfumantes de ces dégradations au cours de la durée de leur entière consommation, on y incorpore généralement des filtres actifs contre les UV (encore appelés « filtres UV » dans le présente demande) et un système antioxydant afin de garantir leur stabilité.

Il a toujours été décrit dans l'art antérieur que l'utilisation de deux filtres UV ne permet pas de protéger un parfum d'une dégradation par les UV. Aussi, il a été proposé des mélanges ternaires ou quaternaires de filtres UV afin de garantir la stabilisation des parfums.

Par exemple, il a été montré dans la demande FR 2 916 346 que la couleur et l'odeur d'une eau de toilette comprenant 1% en poids d'une mélange de 2-(4-diéthylamino-2-hydroxybenzoyl)-benzoate de n-hexyle et de Ethylhexyl Methoxycinnamate 35/65 se dégrade aux UV et à la chaleur, si bien qu'il est nécessaire d'y ajouter un troisième filtre UV du type butyl-méthoxy-dibenzoyl-méthane pour obtenir une protection efficace du produit vis-à-vis des UV.

De même, la couleur et l'odeur d'une eau de toilette comprenant 1% en poids d'éthylhexyl-méthoxycinnamate et 0,3% en poids de butyl-méthoxy-dibenzoyl-méthane se dégradent aux UV et à la chaleur. C'est pourquoi il a été proposé dans la demande WO 2006/005846, d'ajouter à l'éthyl-hexyl-méthoxycinnamate et au butyl-méthoxy-dibenzoyl-méthane un troisième filtre, l'éthyl-hexyl-salicylate, pour protéger les compositions cosmétiques d'une décoloration lorsqu'elles sont exposées aux UV.
Les demandes FR 2 916 347 et FR 2 923 385 ont aussi décrit des mélanges de filtres comprenant un cinnamate et un dibenzoylméthane pour garantir la stabilité de la couleur et de l'odeur d'une eau de toilette. Ces mélanges quaternaires comprennent un cinnamate, un dibenzoylméthane et un dérivé salicylate, en combinaison avec soit un dérivé de hydroxyaminobenzophénone, soit un dérivé de benzotriazole. L'éthyl-hexyl-salicylate présente cependant l'inconvénient d'être odorant: il dégage une note grasse, pyrogénée, plastique, caoutchouc, si bien que son utilisation pour la stabilisation de solutions parfumantes n'est pas satisfaisante ; suivant le pourcentage auquel il est introduit pour cette utilisation, il peut impliquer une dégradation de l'authenticité du parfum.

Enfin, il a été proposé d'ajouter à un mélange de filtres UV comprenant un cinnamate et un dibenzoylméthane, un composé organique comprenant un groupe nitroxyle ou hydroxylamine tel que le tris-tétra-méthylhydroxypiperidinol (commercialisé sous la référence du Tinogard Q) pour protéger des tissus, des compositions cosmétiques et des produits d'entretien ménager des effets de la lumière, de la chaleur et de l'oxydation (demande de brevet WO 2005/042828).

Malheureusement les additifs stabilisants préconisés dans les demandes de brevet de l'art antérieur contiennent des quantités élevées de filtres UV, qui sont des matières premières coûteuses, et qui ont tendance à dénaturer la couleur ou le parfum de la solution parfumante.

On a également cherché à augmenter la quantité de filtres dans les parfums afin de protéger les produits vis-à-vis des rayons UV ; cependant, cette voie n'a pas abouti parce qu'à des concentrations plus élevées, le film de parfum vaporisé sur la peau laisse une sensation de gras et que des contraintes réglementaires imposent des limites maximales du taux d'utilisation de chaque filtre UV.

### But de l'invention

Il subsiste donc le besoin de proposer de nouveaux stabilisants de produits parfumants ne présentant pas les inconvénients des stabilisants de l'art antérieur, et notamment des mélanges stabilisants qui ne modifient pas les propriétés organoleptiques comme l'odeur et la couleur du produit parfumant, tout en procurant une stabilisation suffisante du produit exposé à la lumière du jour ou à des différences de température.

Des stabilisants moins coûteux qui ne modifient pas les propriétés organoleptiques comme l'odeur et la couleur des parfums et des eaux de toilette, tout en procurant une stabilisation suffisante du produit exposé à la lumière du jour ou à des différences de température, sont donc recherchés.

La demanderesse a découvert de manière surprenante que cet objectif pouvait être atteint en associant deux filtres UV particuliers et une huile non volatile, de préférence polaire.

Contrairement à l'enseignement de l'art antérieur qui suggère d'utiliser au moins trois filtres UV pour obtenir une stabilisation des solutions parfumantes suffisante vis-à-vis de l'action des UV, les inventeurs ont trouvé qu'il est possible contre toute attente de n'utiliser que deux filtres UV, dans une eau de toilette tout en garantissant une stabilité du parfum et de la couleur suffisante. Il est aussi possible d'incorporer des quantités de filtres UV moindres que celles préconisées dans l'art antérieure tout en garantissant une stabilité suffisante. Les inventeurs ont trouvé de manière surprenante que certaines huiles non volatiles permettent d'augmenter l'efficacité des filtres UV dans une solution parfumante. Ces huiles améliorent en effet la solubilité des filtres UV, si bien qu'il est possible d'en incorporer en plus faible quantité tout en garantissant une stabilisation du produit suffisante. Les inventeurs ont découvert qu'il est même possible de n'utiliser que deux filtres UV pour stabiliser efficacement une solution parfumante exposée aux UV.

### Résumé de l'invention

L'invention se rapporte à une solution parfumante comprenant au moins un alcool volatil, un parfum et un mélange stabilisant de ladite solution constitué d'un filtre UV choisi parmi les dérivés du cinnamate, d'un filtre UV choisi parmi les dérivés du dibenzoylméthane, et d'au moins une huile non volatile soluble dans ledit alcool et solvant desdits filtres, le mélange stabilisant étant en quantité suffisante pour protéger l'odeur de la solution parfumante d'une dégradation par les rayons UV.
Cette solution parfumante comprend au plus deux filtres UV.
L'association de filtres UV conforme à l'invention présente l'avantage d'être moins colorée que d'autres associations de filtres connues.

L'invention se rapporte également à l'utilisation dans une solution parfumante, de l'association d'un filtre UV choisi parmi les dérivés du cinnamate, d'un filtre UV choisi parmi les dérivés du dibenzoylméthane et d'au moins une huile non volatile, comme agent stabilisant des propriétés organoleptiques, en particulier de l'odeur et éventuellement de la couleur, de ladite solution vis-à-vis des rayons UV.

L'invention se rapporte également à un procédé de stabilisation des propriétés organoleptiques, en particulier la couleur et/ou l'odeur d'une solution parfumante, caractérisé par le fait qu'il consiste à incorporer dans la solution l'association d'un filtre UV choisi parmi les dérivés du cinnamate, d'un filtre UV choisi parmi les dérivés du dibenzoylméthane et d'au moins une huile non volatile.

L'invention a encore pour objet un procédé cosmétique pour parfumer des matières kératiniques, notamment la peau, les lèvres, les cheveux, le cuir chevelu, les cils, les sourcils ou les ongles, comprenant l'application sur les matières kératiniques de la solution parfumante telle que définie ci-dessus.

### Description détaillée de l'invention

L'invention a pour premier objet une solution parfumante comprenant au moins un alcool volatil, un parfum et un mélange stabilisant de ladite solution constitué
- d'un filtre UV choisi parmi les dérivés du cinnamate,
- d'un filtre UV choisi parmi les dérivés du dibenzoylméthane, et
- d'au moins une huile non volatile soluble dans ledit alcool et solvant desdits filtres,
   le mélange stabilisant étant avantageusement en quantité suffisante pour protéger l'odeur de la solution parfumante d'une dégradation par les rayons UV.

L'invention concerne également une solution parfumante comprenant 40 à 95% en poids d'au moins un alcool volatil, un parfum, à titre de filtre UV, le mélange d'un filtre UV choisi parmi les dérivés du cinnamate et d'un filtre UV choisi parmi les dérivés du dibenzoylméthane, et au moins une huile choisie parmi les mono- et di-esters aliphatiques, les esters aromatiques non hydroxylés, les carbonates aliphatiques et les silicones phénylés. L'huile non volatile représente de préférence de 0,001% à 1% en poids, de préférence de 0,05% à 0,5% en poids de la solution parfumante.

Par solution parfumante, on entend une composition contenant un alcool, de préférence à titre d'ingrédient majoritaire, dans lequel est solubilisé un parfum laissant après application sur les matières kératiniques, un parfum. Les émulsions eau-dans-huile et huile-dans-eau sont exclues de cette définition. La solution parfumante est avantageusement translucide ou transparente, ce qui rend d'autant plus nécessaire sa protection vis-à-vis de la lumière visible et des rayons UV.
La solution parfumante selon l'invention peut se présenter sous forme d'eau fraîche, d'eau de toilette, d'eau de parfum, de lotion après-rasage, de lotion démaquillante, ou d'eau de soin.

La solution parfumante conforme à la présente invention contient au moins un alcool volatil.

Par alcool volatil, on peut entendre, au sens de l'invention, tout composé comprenant au moins une fonction alcool ayant notamment une pression de vapeur, à 25°C et 0,1 MPa, allant de 0,13 à 40 000 Pa, de préférence de 1,3 à 13 000 Pa, de préférence encore de 1,3 à 8 000 Pa, par exemple supérieure à 2000 Pa.
L'alcool volatil conforme à la présente invention est choisi de préférence parmi les mono-alcools ayant de 1 à 5 atomes de carbone et en particulier parmi le méthanol, l'éthanol, le propanol, l'isopropanol, le n-butanol, l'isobutanol le t-butanol, et leurs mélanges.

Le ou les alcools volatils sont présents de préférence dans des quantités allant de 40 à 95%, et plus préférentiellement dans des quantités allant de 55 à 80% en poids par rapport au poids total de la solution.

Selon un mode de réalisation préféré, la solution contient de l'eau dans une quantité allant de 0,01 % à 20 %, de préférence de 0,1 % à 15 %, et plus préférentiellement, de 0,2 % à 12 % en poids par rapport au poids total de la solution.

On entend par parfum une substance odorante ou un mélange de substances odorantes qui s'évaporent à 25°C. Chaque parfum présente ce que l'on appelle une note de tête qui est l'odeur diffusant en premier lors de l'application du parfum ou lors de l'ouverture du récipient le contenant, une note de coeur ou corps qui correspond au parfum complet (émission pendant quelques heures après la note de tête) et une note de fond qui est l'odeur la plus persistante (émission pendant plusieurs heures après la note de coeur). La persistance de la note de fond correspond à la rémanence du parfum.
Le parfum peut par exemple être choisi parmi des composés dont le nom INCI figurant sur la liste des ingrédients de la solution parfumante proposée à la vente est « Parfum ». Un parfum est un composé au moins partiellement volatil à température ambiante, dont on détecte l'odeur.
Par parfum, on entend encore tout composé organique susceptible de parfumer la peau les cheveux, le cuir chevelu, les lèvres ou les ongles.

La quantité de parfum, encore appelé concentré, sera plus préférentiellement de 3 à 50 % en poids, mieux de 5 à 30%, encore mieux 10 à 20% en poids par rapport au poids total de la solution.
Comme parfum, on peut utiliser dans la solution parfumante de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme parfum d'origine synthétique, on peut citer par exemple l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle, le benzyléthyléther, les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal, les ionones comme l'alpha-isométhylionone, et la méthylcédrylcétone, l'anéthol, le citronellol, l'eugénol, l'isoeugénol, le géraniol, le linalol, le phényléthylalcool, le terpinéol, les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On utilise de préférence comme parfum, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le 25 linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges

Le parfum peut également contenir du triéthyl citrate en tant que solvant et/ou diluant.

Le parfum représente généralement de 5 à 40%, de préférence de 10 à 30 % en poids du poids de la solution parfumante de l'invention.

Le mélange stabilisant selon l'invention comprend un filtre UV choisi parmi les dérivés du cinnamate et un filtre UV choisi parmi les dérivés du dibenzoylméthane. Par "filtre UV", on entend tout composé absorbant un rayonnement UV dans la gamme de longueurs d'onde allant de 280 nm à 400 nm.

La solution parfumante est avantageusement exempte d'un filtre UV choisi parmi les dérivés du salicylate.

Parmi les filtres UV dérivés du cinnamate, on peut citer notamment de manière non limitative: l'éthyl-2-hexyl-p-methoxycinnamate, l'isopropyl-p-methoxycinnamate, Isoamyl Methoxycinnamate, le Cinoxate (2-éthoxyéthyl-p-methoxycinnamate), Diéthanolamine Methoxycinnamate, Glyceryl Ethyl-2-hexanoate Di-p-methoxycinnamate, [4-bis(trimethylsiloxy)methylsilyl-3-methylbutyl]-3,4,5,-trimethoxycinnamate.
Parmi les dérivés cinnamates mentionnés ci-dessus, on utilisera tout particulièrement l'éthyl-2-hexyl-p-methoxycinnamate encore appelé Ethylhexyl Methoxycinnamate ou Octyl Methoxycinnamate proposé à la vente sous les dénominations commerciales PARSOL MCX de la Société DSM NUTRITIONAL PRODUCTS, UVINUL MC 80 de la société BASF, et Uvinul A+B de la société BASF.

Le filtre UV choisi parmi les dérivés du cinnamate peut représenter de 20 à 90% en poids, de préférence de 30 à 75% en poids, du poids du mélange stabilisant constitué des filtres UV et de l'huile.

Le filtre UV choisi parmi les dérivés du cinnamate représente avantageusement de 0,3 à 0,4% en poids du poids de la solution parfumante.

Parmi les filtres UV dérivés du dibenzoylméthane, on peut notamment citer, de manière non limitative: le 2-méthyldibenzoylméthane, le 4-méthyldibenzoylméthane, le 4-isopropyldibenzoylméthane, le 4-tert-butyldibenzoylméthane, le 2,4-diméthyldibenzoylméthane, le 2,5-diméthyldibenzoylméthane, le 4,4'-diisopropyldibenzoylméthane, le 4,4'-diméthoxydibenzoylméthane, le 4-tert-butyl-4'-méthoxydibenzoylméthane, le 2-méthyl-5-isopropyl-4'-méthoxydibenzoylméthane, le 2-méthyl-5-tert-butyl-4'-méthxydibenzoylméthane, le 2,4-diméthyl-4'-méthoxydibenzoylméthane, le 2,6-diméthyl-4-tert-butyl-4'-méthoxydibenzoylméthane. Parmi les dérivés du dibenzoylméthane mentionnés ci-dessus, on utilisera tout particulièrement le 4-(ter-butyl)-4'-méthoxy dibenzoylméthane encore appelé Avobenzone ou Butyl Methoxy Dibenzoylmethane (BMDBM) proposé à la vente sous les dénominations commerciales de PARSOL 1789 de la Société DSM NUTRITIONAL PRODUCTS, ou EUSOLEX 9020 de la société MERCK.

Le filtre UV choisi parmi les dérivés du dibenzolyméthane peut représenter de 5 à 75% en poids, de préférence de 10 à 55% en poids, du poids du mélange stabilisant constitué des filtres UV et de l'huile.

Le filtre UV choisi parmi les dérivés du dibenzolyméthane représente avantageusement de 0,05 à 0,1% en poids du poids de la solution parfumante. La quantité totale de filtres UV dans la solution parfumante représente de préférence de 0,005 à 5% en poids, de manière encore préférée de 0,1% à 1% en poids, du poids de la solution parfumante.

Le mélange stabilisant peut comprendre outre des filtres UV, au moins une huile non volatile.
Par huile, on entend, au sens de l'invention, un corps gras, non soluble dans l'eau, et liquide à 25°C et 0,1 MPa. Par huile non volatile on entend toute huile ayant une pression de vapeur, à 25°C et 0,1 MPa, non nulle inférieure à 2,6 Pa, de préférence inférieure à 0,13 Pa. Au sens de la présente invention, une huile non volatile n'est pas un parfum et n'est pas un filtre UV organique.

L'huile non volatile est de préférence soluble à 25°C dans l'alcool, ingrédient dont la proportion massique est majoritaire dans la solution parfumante. L'huile est de préférence dénuée d'une odeur. L'huile non volatile est de préférence incolore.

L'huile non volatile est de préférence miscible dans l'alcool et solvant desdits filtres, de préférence sans apport de chaleur. On peut évaluer la solubilité de l'huile non volatile dans l'alcool par le protocole de mesure de la miscibilité suivant. Dans un bécher, on pèse l'alcool sélectionné (80% en poids) puis on ajoute au bêcher l'huile sélectionnée (20% en poids). On agite pendant 5 minutes et puis on conditionne le tout dans un pilulier de 120 ml. On laisse reposer pendant 24 heures à 25°C.

Après 24 heures de repos, si le mélange est visuellement limpide et homogène, on considère que l'huile est miscible dans l'alcool.

L'huile est de préférence polaire, en ce sens qu'elle contient au moins un, de préférence au moins deux atomes d'oxygène ou des doubles liaisons conjuguées.

Selon un mode de mise en oeuvre, l'huile est une huile hydrocarbonée comprenant au plus un cycle aliphatique. L'huile est de préférence une huile aliphatique, c'est-à dire qu'elle n'est pas aromatique au sens où elle ne contient pas un système cyclique respectant la règle d'aromaticité de Hückel.
Par aliphatique, on entend un composé non aromatique. Par ester, éther, alcool ou acide aliphatique, on entend un composé constitué d'atomes de carbone, d'hydrogène et respectivement d'un groupe COO, COC, OH ou COOH. Par ester aliphatique hydroxylé, on entend un composé hydrocarboné comprenant un group COO et au moins un groupe OH, de préférence un seul groupe OH.

Selon un autre mode de mise en oeuvre, l'huile est une huile siliconée comprenant au moins un groupe carboné aromatique.

Par ester aliphatique, on entend un composé constitué d'atomes de carbone, d'atomes d'hydrogène et d'au moins un groupe COO. Par monoester, on entend un composé comprenant un groupe COO, et par diester, on entend un composé comprenant deux groupes COO.
Par ester hydroxylé, on entend un composé comprenant au moins un groupe COO et au moins un groupe OH.
Dans un autre mode de réalisation, on préfère un ester choisi parmi les monoesters et les diesters aliphatiques. Les esters hydroxylés sont de préférence non aromatiques.

L'huile non volatile est de préférence choisi parmi les mono- et di-esters aliphatiques, les esters aromatiques non hydroxylés, les carbonates aliphatiques et les silicones phénylés.

Comme huiles utilisables dans la solution parfumante de l'invention, on peut citer par exemple :
- les mono-et di-esters aliphatiques, notamment i) les mono-esters d'un acide carboxylique aliphatique linéaire ou ramifié, saturé ou insaturé, de préférence saturé, comprenant de 8 à 20 atomes de carbone, et d'un mono-alcool aliphatique comprenant de 3 à 20 atomes de carbone, ii) les di-esters aliphatiques d'un di-acide aliphatique carboxylique comprenant de 4 à 10 atomes de carbone et d'un mono-alcool,
- les mono-esters de l'acide benzoïque et d'un alcool aliphatique comprenant de 8 à 20 atomes de carbone, le benzoate d'éthyle-2-hexyle, le benzoate d'octyl-2-dodécyle, le benzoate d'isostéaryle, le C12-C15 alkylbenzoate,
- les di-alkyl-carbonates dont les groupes alkyles contiennent de 8 à 18 atomes de cabone tels que le dicaprylyl-carbonate, le di-(éthyl-2-hexyl)-carbonate,
- les mono ou di-esters aliphatiques hydroxylés tels que i) les esters d'un mono ou di-acide carboxylique aliphatique hydroxylé comprenant de 3 à 20 atomes de carbone, et d'un mono-alcool aliphatique aliphatique comprenant de 6 à 20 atomes de carbone, par exemple le lactate d'isostéaryle, l'hydroxystéarate d'octyle, l'hydroxystéarate d'octyldodécyle, le lactate de cétyle, le lactate de myristyle, le diisostéaryl-malate, ou ii) les mono et di-esters aliphatiques de polyol, en particulier de diols et de triols, tels que les esters d'un mono acide carboxylique aliphatique comprenant de 3 à 20 atomes de carbone, et d'un diol ou d'un triol aliphatique comprenant de 3 à 20 atomes de carbone,
- les alcools aliphatiques saturés ou insaturés ayant de 8 à 26 atomes de carbone, comme l'octyldodécanol, l'octyldécanol, le 2-butyloctanol, le 2-hexyldécanol, le 2-undécylpentadécanol,
- les acides mono-carboxyliques aliphatiques saturés ou insaturés ayant de 7 à 29 atomes de carbone tels que l'acide oléique, l'acide linoléique, l'acide linolénique ou l'acide isostéarique,
- les huiles de silicone comportant au moins un groupement alcoxy ou phényle, pendant ou en bout de chaîne siliconée, ayant de 2 à 24 atomes de carbone, notamment la phényltriméthicone, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes;
- leurs mélanges.

Parmi les mono- et di-esters aliphatiques, on préfère les esters comprenant de 10 à 25 atomes de carbone, de préférence de 14 à 22 atomes de carbone, par exemple les esters de l'acide isononanoïque tels que l'isononanoate d'isononyle, l'isononanoate d'isodécyle, l'isononanoate de décyl-2-héxyle, l'isononanoate d'isostéaryle, l'isononanoate de cétéaryle, l'isononanoate de tridécyle.

On peut citer aussi le myristate d'isopropyle, le palmitate d'éthyl-2-hexyle, le stéarate d'octyl-2-dodécyle, l'érucate d'octyl-2-dodécyle, l'isostéarate d'isostéaryle, le stearoylstearate d'octyl dodecyl, le palmitate d'isostéaryle, le stéarate d'isocétyle, le myristate d'octyldodécyle, le trilinoléate de triisostéaryle, le néodécanoate d'octyldodécyle, l'octyldodécyl octanoate, le stéarate d'isobutyle, la néopentanoate d'isodécyle, le néopentanoate d'octyldodécyle, l'iso-stéarate d'éhyl-2 héxyle, l'isostéarate de butyle, le palmitate d'isopropyle, l'heptanoate de stéaryle, le stéarate d'isopropyle, le néopentanoate d'isostéaryle, l'isostéarate d'isopropyle, l'octanoate de cétyle (ou octanoate de palmityle), le stéarate de butyle, le laurate d'hexyle (ou dodécanoate d'hexyle), le laurate d'éthyle, l'oléate de décyle, l'oléate d'oléyle, le myristate de myristyle, le diméthyl-octanoate d'hexyldécyle, l'isostearate d'isocétyle, le myristate de héxyl-2-décyle, le palmitate de heptyl-2-undécyle, le 2-éthyl-hexanoate de cétyle. Parmi les esters de d'acides dicarboxyliques, on peut encore citer le sébaçate de di-(éthyl-2-hexyle), le sébaçate de di-isopropyle, le succinate de di-(éthyl-2-hexyle), l'adipate de di-(héxyl-2-décyle), l'adipate de di-(heptyl-2-undécyle).

Parmi les mono et di-esters de polyol, on peut citer les di-esters d'alkylèneglycol avec un acide aliphatique ayant de 6 à 20 atomes de carbone, tel que le dioctanoate de propylène glycol, le diheptanoate de néopentylglycol et le diisononanoate de diéthylèneglycol; le butylène glycol dicaprate/dicaprylate, le propylene glycol dicaprate/dicaprylate, le di-caprate de néopentyl glycol, le diéthylène glycol diisononanoate, le di-iso-stéarate de propylene glycol, le propylène glycol dipélargonate, le dioctanoate de propylène glycol, le di hepatnoate de néopentyl glycol, le tripropylène glycol dipivalate ; et les mono-alkanoates de glycéryle tel que l'heptanoate de glycéryle, l'octanoate de glycéryle, et le décanoate de glycéryle.

Selon un mode de mise en oeuvre de l'invention, l'huile non volatile est un ester aliphatique non volatil comprenant de 10 à 25 atomes de carbone, de préférence de 14 à 22 atomes de carbone. L'ester aliphatique peut être choisi parmi des esters non hydroxylés d'acides aliphatiques monocarboxyliques ou dicarboxyliques en C2 à C18 et d'alcools choisis parmi les mono-alcools en C2 à C20 et les polyols en C2 à C8.

L'huile non volatile représente de préférence de 1 à 30% en poids, de préférence encore de 10 à 20% en poids du poids du mélange stabilisant, notamment 15% en poids environ du mélange stabilisant, constitué des filtres et de l'huile.

L'huile non volatile représente de préférence de 0,001% à 1% en poids, de préférence de 0,05% à 0,5% en poids de la solution parfumante.

Selon un mode de réalisation, le mélange stabilisant comprend de préférence au plus deux filtres UV. Un mélange stabilisant préféré est constitué du méthoxycinnamate d'octyle, du Butyl Methoxy Dibenzoylmethane et d'une huile non volatile choisie dans le groupe comprenant le diisostéaryl malate, la phényltrimethicone, le butylène glycol dicaprylate/dicaprate, le C₁₂-C₁₅ alkyl benzoate et l'isonanoate d'isononyle.

Un mélange stabilisant préféré se compose de méthoxycinnamate d'octyle, de butyleméthoxy dibenzoylméthane et le butylène glycol dicaprylate / dicaprate. L'octyl méthoxycinnamate représente avantageusement de 60 à 80% en poids du mélange. Le butyle méthoxy-dibenzoylméthane représente avantageusement de 10 à 20% en poids du mélange, et le butylène glycol dicaprylate / dicaprate représente avantageusement de 10 à 20% en poids du mélange stabilisant.

Le mélange stabilisant représente de 0,01% à 10%, de préférence de 0,1% à 5% en poids, de préférence encore de 0,05 à 3% en poids, et plus préférentiellement de 0,1 à 2% en poids, du poids de la solution parfumante.

La solution selon l'invention peut comprendre en outre au moins une matière colorante comme les colorants liposolubles, les colorants hydrosolubles ou solubles dans une solution hydroalcoolique.

L'association de filtres UV conforme à l'invention joue également un rôle de protection vis-à-vis de ces colorants, afin d'éviter tout changement de couleur lié soit directement à la réaction de la lumière ou de la température sur ces colorants ou l'interaction des parfums ou de ses produits dérivés issus de dégradation sur lesdits colorants.

Les colorants hydrosolubles ou solubles dans une solution hydroalcoolique sont par exemple: le caramel, Yellow 5, Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (CI 15850, CI 75470), Ext. Violet 2, Red 6-7, Ferric Ferrocyanide, Ultramarines, Acide Yellow 3 / Yellow 10, Acid Blue 3, Yellow 10. Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou.

Les colorants représentent généralement de 0,01 à 1%, de préférence de 0,05 à 0,5%, en poids du poids de la solution parfumante.

La solution parfumante de l'invention peut comprendre, en outre, tout additif usuellement utilisé dans le domaine des parfums choisi notamment parmi les antioxydants. Parmi les antioxydants, on peut citer par exemple l'acide ascorbique, le di-tert-butyl-p-hydroxytoluène (encore appelé BHT ou 2,6-di-tert-butyl-p-crésol), le BHA (tert-butyl-4-hydroxyanisole), les tocophérols comme la vitamine E, les dérivés du tocophérol tels que l'acétate de tocophéryle, l'acide gallique et ses dérivés.

La solution selon l'invention peut être fabriquée par les procédés connus, généralement utilisés dans le domaine des compositions parfumées.

Les solutions parfumantes selon l'invention peuvent être conditionnées sous forme de flacons. Elles peuvent également être appliquées sous forme de fines gouttelettes au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur.

Les solutions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane.
Les solutions parfumantes selon l'invention se présentent notamment sous forme de solutions hydroalcooliques.

La présente invention a encore pour objet l'utilisation d'un mélange stabilisant tel que décrit précédemment et comprenant un filtre UV dérivé du cinnamate, un filtre UV dérivé du dibenzoylméthane, et au moins une huile choisie parmi le butylène glycol dicaprylate/dicaprate, la phényltriméthicone, le di-isostéaryl-malate, l'isononanoate d'isononyle et le dicapryl-carbonate pour protéger une solution parfumante d'une dégradation de son odeur par les rayons UV.

La solution parfumante peut contenir en outre au moins un colorant, auquel cas le mélange stabilisant protége la solution parfumante d'une dégradation de sa couleur par les rayons UV.

Le mélange stabilisant représente de préférence de 0,01% à 10% en poids, de préférence de 0,1% à 5% du poids de la solution parfumante.

Les caractéristiques décrites en relation avec la solution parfumante sont applicables à l'utilisation d'un mélange stabilisant selon l'invention.

D'autres buts, caractéristiques et avantages de l'invention apparaîtront clairement à l'homme de l'art suite à la lecture des exemples qui sont donnés seulement à titre d'illustration et ne sauraient donc en aucune façon limiter la portée de l'invention. Les exemples font partie intégrante de la présente invention et ont ainsi une portée générale.

D'autre part, dans les exemples ci-dessous, tous les pourcentages sont donnés en poids, la température est exprimée en degré Celsius sauf indication contraire, et la pression est la pression atmosphérique.

### EXEMPLES

### A- Préparation des solutions parfumantes

On a préparé des solutions parfumantes, encore appelées fragrances, de composition suivante:

**Tableau 1**

| | Fragrance N° 1 | Fragrance N° 2 | Fragrance N° 3 |
|---|---|---|---|
| Alcool à 96,2% vol | Qsp 100 | Qsp 100 | Qsp 100 |
| Concentré | 11,00 de concentré n°1 | 17,85 de concentré n°2 | 30,00 de concentré n°3 |
| Eau purifiée | 10,38 | 2,54 | 0,56 |
| colorants | 0,11 | 0,10 | 0,43 |
| BHT | 5 x 10⁻³ | 5 x 10⁻³ | 1 x 10⁻² |

Le concentré n°1 se composait majoritairement d'hédione, le concentré n°2 se composait majoritairement d'essence de rose, et le concentré n°3 se composait majoritairement d'absolu de jasmin. Dans chacune de ces fragrances on a incorporé un mélange de filtres et d'huile non volatile selon l'invention ayant l'une des quatre compositions suivantes :

**Tableau 2**

| | Mélange 1 | Mélange 2 | Mélange 3 | Mélange 4 |
|---|---|---|---|---|
| | 70% METHOXYCINNAMATE D'OCTYLE | | | |
| | 15% BMDBM* | | | |
| DIISOSTEARYL MALATE | 15% | | | |
| PHENYL TRIMETHICONE | | 15% | | |
| BUTYLENE GLYCOL DICAPRYLATE/ DICAPRATE | | | 15% | |
| ISONONYL ISONONANOATE | | | | 15% |

| | | | | |
|---|---|---|---|---|
| ** BMDBM 4-(ter-butyl)-4'-méthoxy dibenzoylméthane* | | | | |

Dans un bécher on a mélangé l'éthanol et l'eau sous agitation Ystral à froid pendant 5 min. On a ensuite ajouté les colorants sous la forme d'une solution dans l'éthanol ou dans l'eau selon les cas, au mélange hydroalcoolique, et laissé sous agitation pendant 5 minutes.

Dans une capsule, on a mélangé l'anti-oxydant BHT, le BMDBM, le methoxycinnamate d'octyle et l'huile non volatile à 70°C sous agitation magnétique, en faisant varier la quantité du mélange dans la fragrance entre 0,1 et 5% en poids.
On a ensuite laissé refroidir le mélange et ajouté le parfum sous agitation magnétique. Le contenu de la capsule a été ensuite mélangé à la solution colorée hydroalcoolique sous agitation pendant 10 min, conditionné dans un atomiseur parfum pour tester son aptitude à être pulvérisé, dans des piluliers de 30 ml remplis à 25 ml pour les stabilités étuves, puis dans des flacons verres de 60 ml pour le SUN TEST et l'expertise olfactive.

### B - Stabilité et évaluation sensorielle des solutions parfumantes

On a ensuite évalué la stabilité à la chaleur, la stabilité au froid et la stabilité aux rayonnements UV de chacune des trois fragrances à laquelle est ajouté un des quatre mélanges décrits précédemment, selon les protocoles suivants.

### Stabilité de l'homogénéité de la solution en étuve à 4°C et 45°C

Les piluliers de 30 ml, remplis à 25 ml d'une fragrance, sont placés dans une étuve à 45°C et dans un réfrigérateur à 4°C pendant 3 mois. La solution est stable (résultat conforme) si aucun déphasage ni aucun trouble ou dépôt significatif n'apparaît.

### Stabilité de la couleur et de l'odeur aux UV

On a mesuré les coordonnées L*a*b* de la couleur du mélange placé dans un flacon de 60 ml en verre rempli à 55 ml d'une fragrance, avant irradiation à l'aide d'un spectro-colorimètre Minolta 3600D CM, POS 0118.
Les flacons, contenant chacun une fragrance, sont positionnés debout ce qui permet une exposition totale aux UV du flacon et cela pendant 12 heures dans les conditions suivantes.

L'irradiation de chaque échantillon est réalisée dans un appareil Suntest™, modèle CPS. La source UV utilisée est une lampe Xénon émettant entre 300 et 800 nm à une puissance de 765 W/m². La lampe xénon est associée à un filtre "short cut-off" en quartz combiné à un verre "UV Special Glass". L'échantillon est maintenu à une température de 25°C (en utilisant des plateaux de refroidissement et/ou de la climatisation).
Au bout de 12 heures d'irradiation, on a mesuré à nouveau les coordonnées L*a*b* de la solution parfumante. La couleur de la solution est considérée stable (résultat conforme) si l'écart entre les deux mesures dans des conditions de mesure standard (Illuminant D₆₅ et Observateur à 10°), exprimé par le delta E, est inférieur à 5, de préférence inférieur à 1.

L'odeur de la solution parfumante avant et après exposition à un rayonnement UV dans les conditions décrites précédemment est également évaluée par un expert olfactif.

Le résultat du test est jugé conforme si l'expert olfactif ne détecte aucune différence significative d'intensité de l'odeur après exposition aux rayonnements UV.

### Sensation de gras laissé sur la peau (évaluation sensorielle)

La fragrance est appliquée dans le cou par pulvérisation à l'aide d'un atomiseur, et l'on a cherché à détecter la sensation de gras résiduel restant sur la peau, une fois l'alcool et l'eau évaporés.
L'évaluation repose sur la sensation de gras résiduelle sur la peau, ou sur des observations visuelles (pellicule grasse résiduelle ou vêtements tachés).
Par exemple, une solution contenant la fragrance N°2 et 10% en poids du mélange 2, 3 ou 4 est jugée trop grasse par l'expert.
Le résultat du test est jugé conforme si l'expert ne détecte ni sensation gênante ni observation de type gras.

Les résultats de ces différentes évaluations sont reportés dans les tableaux 3 à 8 ci-dessous. Les mentions entre parenthèses nuancent le résultat obtenu sans en modifier la conformité. Les mentions en *italique* caractérisent des observations qui rendent le résultat non-conforme.

**Tableau 3**

| Fragrance n°2 + X% du mélange | | | Mélange 1 | Mélange 2 | Mélange 3 | Mélange 4 |
|---|---|---|---|---|---|---|
| 0,1 | STABILITE ETUVE | 4°C | Conforme | Conforme | Conforme | Conforme |
| | | 45 °C | Conforme | Conforme | Conforme | Conforme |
| | SUN TEST 12H | | Conforme | Conforme | Conforme | Conforme |
| | EVALUATION SENSORIELLE | | Conforme | Conforme | Conforme | Conforme |
| | TEST OLFACTIF APRES 12H DE SUNTEST | | Note olfactive conforme | Note olfactive conforme | Note olfactive conforme | Note olfactive conforme |
| 0,5 | STABILITE ETUVE | 4°C | Conforme | Conforme | Conforme | Conforme |
| | | 45°C | Conforme | Conforme | Conforme | Conforme |
| | SUN TEST 12H | | Conforme | Conforme | Conforme | Conforme |
| | EVALUATION SENSORIELLE | | Conforme | Conforme | Conforme | Conforme |
| | TEST OLFACTIF APRES 12H DE SUNTEST | | Note olfactive conforme | Note olfactive conforme | Note olfactive conforme | Note olfactive conforme |
| 1 | STABILITE ETUVE | 4°C | Conforme | Conforme | Conforme | Conforme |
| | | 45°C | Conforme | Conforme | Conforme | Conforme |
| | SUN TEST 12H | | Conforme | Conforme | Conforme | Conforme |
| | EVALUATION SENSORIELLE | | Conforme | Conforme | Conforme | Conforme |
| | TEST OLFACTIF APRES 12H DE SUNTEST | | Note olfactive conforme | Note olfactive conforme | Note olfactive conforme | Note olfactive conforme |
| 5 | STABILITE ETUVE | 4°C | Conforme | Conforme | Conforme | Conforme |
| | | 45°C | Conforme | Conforme | Conforme | Conforme |
| | SUN TEST 12H | | Conforme | Conforme | Conforme | Conforme |
| | EVALUATION SENSORIELLE | | Conforme (légèrement gras) | Conforme (légèrement gras) | Conforme (légèrement gras) | Conforme (légèrement gras) |
| | TEST OLFACTIF APRES 12H DE SUNTEST | | Note olfactive conforme (légère perte d'intensité). | Note olfactive conforme (légère perte d'intensité). | Note olfactive conforme (légère perte d'intensité). | Note olfactive conforme (légère perte d'intensité). |

**Tableau 4**

| | | Fragrance N°1 | Fragrance N° 1 + 0,5% Mélange 1 | Fragrance N° 1 + 0,5% Mélange 2 | Fragrance N° 1 + 0,5% Mélange 3 | Fragrance N° 1 + 0,5% Mélange 4 |
|---|---|---|---|---|---|---|
| STABILITES ETUVES | 45°C | Conforme | Conforme | Conforme | Conforme | Conforme |
| | 4°C | Conforme | Conforme | Conforme | Conforme | Conforme |
| SUN TEST 12 H | | *Décoloration totale* | Conforme (légèrement rosé) | Conforme (légèrement rosé) | Conforme (légèrement rosé) | Conforme (légèrement rosé) |
| EVALUATION SENSORIELLE | | - | Conforme (pas d'effet gras sur la peau) | Conforme (pas d'effet gras sur la peau) | Conforme (pas d'effet gras sur la peau) | Conforme (pas d'effet gras sur la peau) |
| TEST OLFACTIF APRES 12H DE SUN TEST | | *Perte d'intensité de la note olfactive* | Note olfactive conforme | Note olfactive conforme | Note olfactive conforme | Note olfactive conforme |

**Tableau 5**

| | | Fragrance N°3 | Fragrance N° 3 + 1% Mélange 1 | Fragrance N° 3 + 1% Mélange 2 | Fragrance N° 3 + 1% Mélange 3 | Fragrance N° 3 + 1% Mélange 4 |
|---|---|---|---|---|---|---|
| STABILITES ETUVES | 45°C | Conforme | Conforme | Conforme | Conforme | Conforme |
| | 4°C | Conforme (très légères volutes) | Conforme | Conforme | Conforme | Conforme |
| SUN TEST 12H | | *Décoloration totale* | Conforme (légèrement décoloré) | Conforme (légèrement décoloré) | Conforme (légèrement décoloré) | Conforme (légèrement décoloré) |
| EVALUATION SENSORIELLE | | - | Conforme (Pas d'effet gras sur la peau) | Conforme (pas d'effet gras sur la peau) | Conforme (pas d'effet gras sur la peau) | Conforme (pas d'effet gras sur la peau) |
| TEST OLFACTIF APRES 12H DE SUN TEST | | *Perte d'intensité de la note olfactive* | Note olfactive conforme | Note olfactive conforme | Note olfactive conforme | Note olfactive conforme |

**Tableau 6**

| | | Fragrance N° 1 + 5% Mélange 1 | Fragrance N° 1 + 5% Mélange 2 | Fragrance N° 1 + 5% Mélange 3 | Fragrance N° 1 + 5% Mélange 4 |
|---|---|---|---|---|---|
| STABILITES ETUVES | 45°C | Conforme | Conforme | Conforme | Conforme |
| | 4°C | Conforme | Conforme | Conforme | Conforme |
| SUN TEST 12 H | | Conforme (léger jaunissement) | Conforme (léger jaunissement) | Conforme (léger jaunissement) | Conforme (léger jaunissement) |
| EVALUATION SENSORIELLE | | Conforme (légèrement gras) | Conforme (légèrement gras) | Conforme (légèrement gras) | Conforme (légèrement gras) |
| OLFACTIF APRES 12H DE SUN TEST | | Note olfactive conforme (légère perte d'intensité). | Note olfactive conforme (légère perte d'intensité). | Note olfactive conforme (légère perte d'intensité). | Note olfactive conforme (légère perte d'intensité). |

**Tableau 7**

| Fragrance N° 2+ 0,5% du mélange de l'invention | | Essai 1 comparatif | Essai 2 | Essai 3 | Essai 4 |
|---|---|---|---|---|---|
| | | Composition du mélange | | | |
| METHOXYCINNAMATE D'OCTYLE | | 82,3 | 70 | 50 | 35 |
| BMDBM | | 17,7 | 15 | 35 | 50 |
| BUTYLENE GLYCOL DICAPRYLATE/DICAPRATE | | 0 | 15 | 15 | 15 |
| STABILITES ETUVES | 45°C | Conforme | Conforme | Conforme | Conforme |
| | 4°C | Conforme | Conforme | Conforme | Conforme |
| SUN TEST 12 H | | *Décoloration* | Conforme | Conforme | Conforme |
| EVALUATION SENSORIELLE | | Conforme | Conforme | Conforme | Conforme |
| OLFACTIF APRES 12H DE SUN TEST | | *Perte d'intensité de la note olfactive* | Conforme | Conforme | Conforme |

**Tableau 8**

| | | Fragrance N°2 seule | Fragrance N°2 + 0,5% Essai 1 comparatif | Fragrance N° 2 + 0.5% Mélange 2 | Fragrance N° 2 + 0.5% Mélange 3 | Fragrance N° 2 + 0.5% Mélange 4 |
|---|---|---|---|---|---|---|
| STABILITES ETUVES | 45°C | Conforme | Conforme | Conforme | Conforme | Conforme |
| | 4°C | *Dépôt blanc* | Conforme | Conforme | Conforme | Conforme |
| SUN TEST 12 H | | *Décoloration totale* | *Décoloration* | Conforme (légèrement décoloré) | Conforme (légèrement décoloré) | Conforme (légèrement décoloré) |
| EVALUATION SENSORIELLE | | - | Conforme | Conforme (pas d'effet gras sur la peau) | Conforme (pas d'effet gras sur la peau) | Conforme (pas d'effet gras sur la peau) |
| OLFACTIF APRES 12H DE SUN TEST | | *Perte d'intensité de la note olfactive* | *Perte d'intensité de la note olfactive* | Conforme | Conforme | Conforme |

| | | | | | | |
|---|---|---|---|---|---|---|
| **cf composition du mélange « Essais 1 comparatif du tableau 7.* | | | | | | |

## Revendications

1. Solution parfumante comprenant 40 à 95% en poids d'au moins un alcool volatil, un parfum, le mélange d'un filtre UV choisi parmi les dérivés du cinnamate et d'un filtre UV choisi parmi les dérivés du dibenzoylméthane, et au moins une huile non volatile soluble dans ledit alcool et solvant desdits filtres choisie parmi les mono- et di-esters aliphatiques, les esters aromatiques non hydroxylés, les carbonates aliphatiques et les silicones phénylées,
**caractérisée en ce qu'**elle comprend au plus deux filtres UV.

2. Solution parfumante selon la revendication 1, **caractérisée en ce que** l'alcool volatil est choisi parmi les mono-alcools ayant de 1 à 5 atomes de carbone et en particulier parmi le méthanol, l'éthanol, le propanol, l'isopropanol, le n-butanol, l'isobutanol le t-butanol, et leurs mélanges.

3. Solution parfumante selon l'une quelconque des revendications précédentes, **caractérisée en ce que** le filtre UV choisi parmi les dérivés du cinnamate est l'éthyl-2-hexyl-p-methoxycinnamate.

4. Solution parfumante selon l'une quelconque des revendications précédentes, **caractérisée en ce que** le filtre UV choisi parmi les dérivés du cinnamate représente de 20 à 90% en poids du mélange de filtres et d'huile.

5. Solution parfumante selon l'une quelconque des revendications précédentes, **caractérisée en ce que** le filtre UV choisi parmi les dérivés du dibenzoylméthane est le 4-tert-butyl-4'-méthoxydibenzoylméthane.

6. Solution parfumante selon l'une quelconque des revendications précédentes, **caractérisée en ce que** le filtre UV choisi parmi les dérivés du dibenzolyméthane représente de 5 à 75% en poids du poids du mélange de filtres et d'huile.

7. Solution parfumante selon l'une quelconque des revendications précédentes, **caractérisée en ce que** l'huile non volatile est choisie parmi l'isononanoate d'isononyle, le butylèneglycol dicaprylate/dicaprate, la phényltriméthicone, le di-isostéaryl-malate, le C12-C15 alkyl-benzoate, le dicaprylcarbonate.

8. Solution parfumante selon l'une quelconque des revendications précédentes, **caractérisée en ce que** l'huile non volatile est le butylèneglycol dicaprylate/dicaprate.

9. Solution parfumante selon l'une quelconque des revendications précédentes, **caractérisée en ce que** l'huile non volatile représente de 0,001% à 1% en poids du poids de la solution parfumante.

10. Solution parfumante selon l'une quelconque des revendications précédentes, **caractérisée en ce que** le mélange de filtres et d'huile représente de 0,01% à 10% du poids de la solution parfumante.

## Patentansprüche

1. Parfümierende Lösung, umfassend 40 bis 95 Gewichts-% wenigstens eines flüchtigen Alkohols, einen Duftstoff, die Mischung aus einem UV-Filter, der aus den Derivaten von Cinnamat ausgewählt ist, und aus einem UV-Filter, der aus den Derivaten von Dibenzoylmethan ausgewählt ist, und wenigstens ein nicht flüchtiges Öl, das in dem Alkohol löslich und Lösungsmittel der Filter ist, das aus den aliphatischen Mono- und Diestern, den nicht hydroxylierten aromatischen Estern, den aliphatischen Carbonaten und den phenylierten Silikonen ausgewählt ist,
**dadurch gekennzeichnet, dass** sie höchstens zwei UV-Filter umfasst.

2. Parfümierende Lösung nach Anspruch 1, **dadurch gekennzeichnet, dass** der flüchtige Alkohol aus den Monoalkoholen mit 1 bis 5 Kohlenstoffatomen und insbesondere aus Methanol, Ethanol, Propanol, Isopropanol, n Butanol, Isobutanol, t-Butanol und deren Mischungen ausgewählt ist.

3. Parfümierende Lösung nach einem der vorhergehenden Ansprüche, **dadurch gekennzeichnet, dass** der UV-Filter, der aus den Derivaten von Cinnamat ausgewählt ist, Ethyl-2-hexyl-p-methoxycinnamat ist.

4. Parfümierende Lösung nach einem der vorhergehenden Ansprüche, **dadurch gekennzeichnet, dass** der UV-Filter, der aus den Derivaten von Cinnamat ausgewählt ist, 20 bis 90 Gewichts-% der Mischung aus Filtern und Öl ausmacht.

5. Parfümierende Lösung nach einem der vorhergehenden Ansprüche, **dadurch gekennzeichnet, dass** der UV-Filter, der aus den Derivaten von Dibenzoylmethan ausgewählt ist, 4-tert-Butyl-4'-methoxydibenzoylmethan ist.

6. Parfümierende Lösung nach einem der vorhergehenden Ansprüche, **dadurch gekennzeichnet, dass** der UV-Filter, der aus den Derivaten von Dibenzoylmethan ausgewählt ist, 5 bis 75 Gewichts-% des Gewichts der Mischung aus Filtern und Öl ausmacht.

7. Parfümierende Lösung nach einem der vorhergehenden Ansprüche, **dadurch gekennzeichnet, dass** das nicht flüchtige Öl aus Isononanoat, Isononyl, Butylenglykol Dicaprylat/Dicaprat, Phenyltrimethicon, Diisostearylmalat, C12-C15 Alkylbenzoat, Dicaprylcarbonat ausgewählt ist.

8. Parfümierende Lösung nach einem der vorhergehenden Ansprüche, **dadurch gekennzeichnet, dass** das nicht flüchtige Öl Butylenglykol Dicaprylat/Dicaprat ist.

9. Parfümierende Lösung nach einem der vorhergehenden Ansprüche, **dadurch gekennzeichnet, dass** das nicht flüchtige Öl 0,001 bis 1 Gewichts-% des Gewichts der parfümierenden Lösung ausmacht.

10. Parfümierende Lösung nach einem der vorhergehenden Ansprüche, **dadurch gekennzeichnet, dass** die Mischung aus Filtern und Öl 0,01 bis 10 % des Gewichts der parfümierenden Lösung ausmacht.

## Claims

1. Perfuming solution comprising from 40 to 95 wt.% of at least one volatile alcohol, a perfume, a mixture of one UV filter selected from the derivatives of cinnamate and one UV filter selected from the derivatives of dibenzoylmethane, and at least one non-volatile oil that is soluble in said alcohol and that solubilizes said filters, wherein said non-volatile oil is selected from aliphatic mono- and diesters, non-hydroxylated aromatic esters, aliphatic carbonates and phenylated silicones, **characterized in that** it comprises at most two UV filters.

2. Perfuming solution according to Claim 1, **characterized in that** the volatile alcohol is selected from the monohydric alcohols having from 1 to 5 carbon atoms and in particular from methanol, ethanol, propanol, isopropanol, n-butanol, isobutanol, t-butanol, and mixtures thereof.

3. Perfuming solution according to any one of the preceding claims, **characterized in that** the UV filter selected from the derivatives of cinnamate is ethyl-2-hexyl-p-methoxycinnamate.

4. Perfuming solution according to any one of the preceding claims, **characterized in that** the UV filter selected from the derivatives of cinnamate represents from 20 to 90 wt.% of the filters and oil mixture weight.

5. Perfuming solution according to any one of the preceding claims, **characterized in that** the UV filter selected from the derivatives of dibenzoylmethane is 4-tert-butyl-4'-methoxydibenzoylmethane.

6. Perfuming solution according to any one of the preceding claims, **characterized in that** the UV filter selected from the derivatives of dibenzoylmethane represents from 5 to 75 wt.% of the filters and oil mixture weight.

7. Perfuming solution according to any one of the preceding claims, **characterized in that** the non-volatile oil is selected from isononyl isononanoate, butylene glycol dicaprylate/dicaprate, phenyltrimethicone, diisostearyl malate, C₁₂-C₁₅ alkyl-benzoate, dicapryl carbonate.

8. Perfuming solution according to any one of the preceding claims, **characterized in that** the non-volatile oil is butylene glycol dicaprylate/dicaprate.

9. Perfuming solution according to any one of the preceding claims, **characterized in that** the non-volatile oil represents from 0.001 to 1 wt.% of the weight of the perfuming solution.

10. Perfuming solution according to any one of the preceding claims, **characterized in that** the filters and oil mixture represents from 0.01 to 10 wt.% of the weight of the perfuming solution.
